# EUROPEAN PATENT SPECIFICATION

(11) **EP 1 971 391 B1**
(45) Date of publication and mention of the grant of the patent: **26.12.2012**
(21) Application number: 06846071.6
(22) Date of filing: 27.12.2006
(51) Int. Cl.: A61M 31/00, A61M 5/158, A61M 25/06

(54) **REMOVABLE SHARPS DEVICE FOR ACCESSING A PORTAL RESERVOIR**
ABNEHMBARE VORRICHTUNG FÜR SPITZE GEGENSTÄNDE FÜR DEN ZUGANG ZU EINEM PORTALRESERVOIR
DISPOSITIF POINTU ET TRANCHANT AMOVIBLE PERMETTANT D'ACCEDER A UN RESERVOIR A ORIFICE

(30) Priority: 28.12.2005 US 318485
(43) Date of publication of application: 24.09.2008
(73) Proprietor: Smiths Medical ASD, Inc., St. Paul, MN 55112 (US)
(72) Inventor: MICHELS, Lester, D., Eden Prairie, MN 55347 (US); BELING, William, L., New Brighton, MN 55112 (US); TRAVIS, Ronald, G., Spring Lake Park, MN 55432 (US)
(74) Representative: Lerwill, John
(86) International application number: PCT/US2006/049358
(87) International publication number: WO 2007/079115

(56) References cited:
- WO-A1-99/62576
- US-A1- 2003 069 546
- US-B1- 6 719 721

## Description

### Field of the Invention

The present invention relates to a device for accessing a portal reservoir, and more particularly a portal access device that combines long term accessing of a portal reservoir with sharps protection.

### Background of the Invention

Long term intravenous therapy to a patient oftentimes requires that a portal reservoir (also may be referred herein as port or portal) be implanted to the patient. The medicament stored in the port is fed slowly to the patient via a catheter connected to the portal. To refill the portal, conventionally a needle is inserted through the skin of the patient into a septum of the portal so that the portal may be refilled with the desired medicament. As with the use of any needle or sharps instruments, there is always a chance that the user, or a bystander, may be accidentally pricked by the contaminated needle when it is removed from the patient. Such accidental injury may well be serious as the tip of the needle is contaminated with the fluid from the patient.

A prior device used for shielding the contaminated tip of the needle is disclosed in U.S. patent 6,613,015, assigned to the assignee of the instant application and marketed under the trade name GRIPPER PLUS. In the '015 patent, a right angle needle for accessing the portal is disclosed. The '015 device has the horizontal portion of its right angle needle embedded in an arm that has a distal portion hingedly connected to uprights of a base. The vertical portion of the needle extends through an opening at the proximal portion of the base. Medicament or fluid is fed to the right angle needle via a catheter connected to the distal end of the horizontal portion of the needle. A well or catch is formed above the main body of the base adjacent the opening so that when the arm is pivotally raised relative to the base and when the needle is fully pulled out of the base, the tip of the needle would flex forward so as to be entrapped in the catch adjacent to the opening. Even though surrounded by a dam, the tip of the needle nonetheless is exposed to the environment and viewable by the patient, and there is a remote chance that contaminated fluid at the tip of the needle may adhere to the well, or may be splattered unnecessarily around the area of the well or beyond. A similar right angle device is disclosed in U.S. patent 6,719,721.

After accessing the portal and refilling it, the device of the '015 patent is removed from the skin of the patient. Any subsequent refilling of the portal requires that a new right angle needle device be used to gain access to the portal. This repeated insertion of a needle to the patient tends to cause discomfort, at the very least, to the patient.

There is therefore a need for a portal access device that may be secured to the patient on a long term basis so as not to subject the patient to repeated inserts by needle sharps.

There is also a need for a sharps protection device having the tip of its needle that, once removed from the patient, will not be exposed to the environment or seen by the patient.

US 2003/0069546, upon which the precharacterising portion of claim 1 is based, discloses apparatus comprising a base having a distal portion and a proximal portion, a cannula attached to a first end of an arm, a second end of said arm hingedly connected to the distal portion of said base so that said arm is pivotable relative to said base at its second end, one opening at an upper surface of said base and another opening at a lower surface of said base provided at the proximal portion of said base wherethrough said cannula passes a bore defined between said one opening at the upper surface of said base and said other opening (44) at the lower surface of said base.

Another prior art system is disclosed in WO 99/62576.

According to the present invention there is provided apparatus comprising a base having a distal portion and a proximal portion, a cannula attached to a first end of an arm, a second end of said arm hingedly connected to the distal portion of said base so that said arm is pivotable relative to said base at its second end, one opening at an upper surface of said base and another opening at a lower surface of said base provided at the proximal portion of said base wherethrough said cannula passes a bore defined between said one opening at the upper surface of said base and said other opening at the lower surface of said base; characterised by a first lock mechanism provided at the distal portion said base and a second lock mechanism provided at the second end of said arm, said first and second lock mechanisms coacting to prevent further movement of said arm relative to said base after the tip of said cannula moves past said other opening at the lower surface of said base and is within said bore.

The present invention has the advantage that, to hide the contaminated tip of the needle or the sharp cannula from the user and also the patient, the needle insertion device of the instant invention is configured such that when the tip of the contaminated needle is withdrawn first from the portal reservoir and then the patient, as soon as it is positioned appropriately in the bore formed in the base through which the sharp needle or cannula extends, the movement of the arm that bears the sharp cannula is stopped and locked into place, so that the tip of the needle would not come into contact with any part of the base, and is not withdrawn above the top surface, or the opening at the top surface, of the base. Locking mechanisms at the end of the arm that hingedly connects to the base and at the uprights to which the arm is hingedly attached cooperatively coact to prevent any further upward and downward movements of the arm, once the tip of the needle is appropriately positioned within the bore.

The present invention will become apparent and the invention itself will be best understood by reference to the following description of the present invention taken in conjunction with the accompanying drawings, wherein:

Fig. 1 is a perspective view of the safety needle inserter device of the instant Invention;

Fig. 2 is a perspective view of a first embodiment of the safety needle inserter of the instant invention;

Fig. 3 is an enlarged view of the interconnection between the base and the arm of the safety needle inserter of the instant invention;

Fig. 4 is another view of the interconnection between the arm and the base of the safety needle inserter of the instant invention;

Fig. 5 is another embodiment of the safety needle inserter of the instant invention showing in particular the location of the tip of the needle, or sharp cannula, positioned within the bore of the base.

Fig. 6 is a side view of another embodiment of the safety needle inserter of the instant invention, with the arm of the inserter shown lying longitudinally along the base and the sharp cannula extending downwardly from the base;

Fig. 7 is another view of the safety needle inserter of the Fig. 6 embodiment showing the sharp cannula having been withdrawn from the opening of the base with the tip residing within the bore of the base and the arm having been pivoted upwardly relative to the base;

Fig. 8 is a front perspective view of the safety needle inserter of Figs. 6 and 7, sans the sharp cannula, for showing the interrelationship between the locking mechanisms at the uprights of the base and the end of the arm hingedly connected to the uprights of the base;

Fig. 9 shows the portal access device of the instant invention in which the safety needle inserter component of the instant invention is superposed over an infuser assembly component of the instant invention;

Fig. 10 is a bottom view of the portal access device of the instant invention showing in particular the underside of the infuser and the safety needle inserter,

Fig. 11 a and Fig. 11 b show the sharp cannula of the safety needle inserter mated axially to the blunt cannula of the infuser and withdrawn from the infuser, respectively; and

Fig. 12a and Fig. 12b are respective views of the portal access device showing the mating of the sharp cannula to the blunt cannula and the removal of the sharp cannula from the blunt cannula, respectively.

### Detailed Description of the Invention

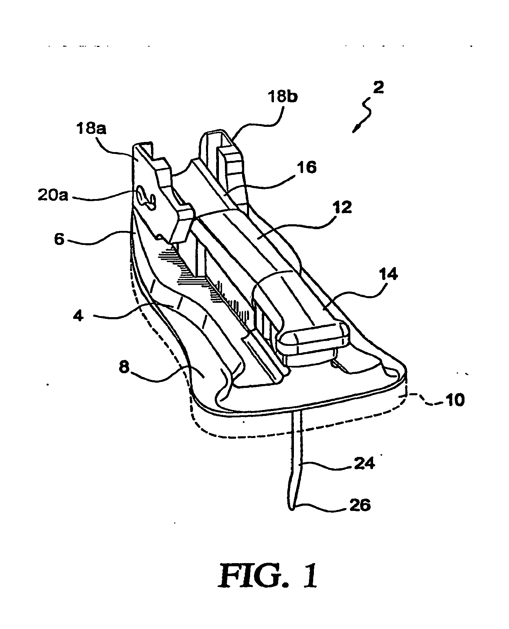

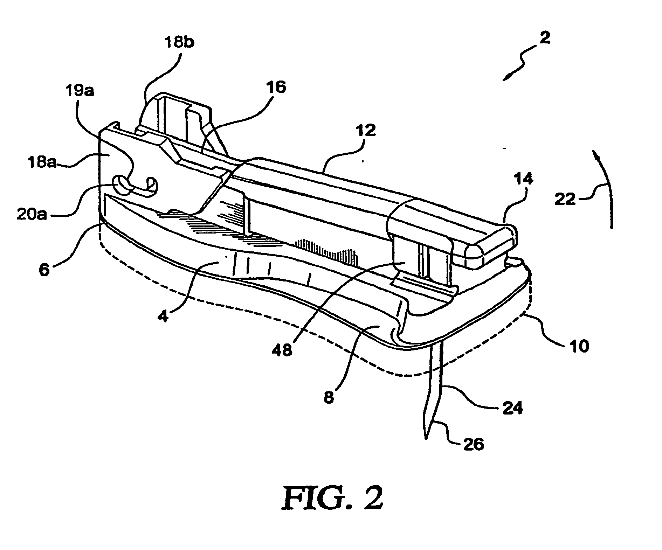

The first embodiment of the safety needle inserter device component of the instant invention is shown in Figs. 1-4. As shown, the safety needle inserter 2 has a base 4 that has a distal portion 6 and a proximal portion 8. A foam pad or layer 10 coated with an adhesive for adhering base 4 to the patient may be mounted to the underside, or the lower surface, of base 4. An arm having a first end 14 and a second end 16 is hingedly connected to two uprights 18a and 18b, respectively, at distal portion 6 of base 4. Two lugs 19, one at each side of second end 16 of arm 12, are movably journaled to the respective guide holes 20a and 20b of the respective uprights 18a and 18b, to thereby hingedly and movably connect arm 12 to base 4. Only lug 19a is shown in Fig. 2. When uplifted in the direction as indicated by directional arrow 22, arm 12 is pivotally moved, via its second end 16, in an upward direction relative to base 4.

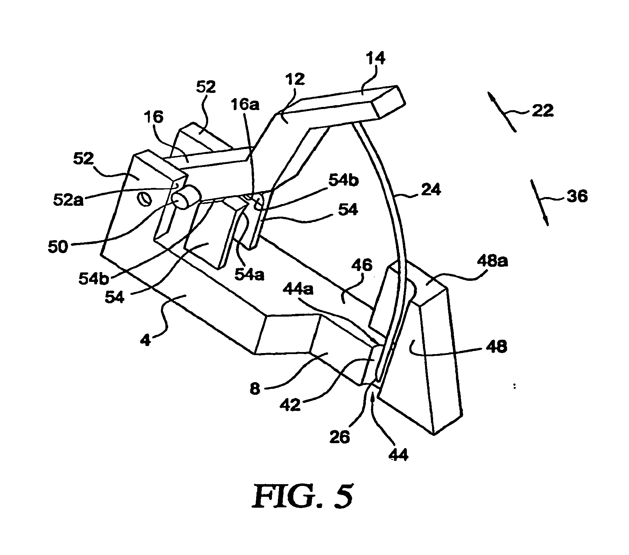

A needle, or sharp cannula 24, is mounted to the underside of arm 12, at its first end 14. As shown in Figs. 1 and 2, when arm 12 lies substantially longitudinally along the length of base 4, needle 24 extends downwardly from base 4 through an opening 44, best shown in the embodiment of Fig. 5. As shown, sharp cannula 24 has a tip 26 that is adapted to insert into the patient and penetrate the septum of a portal reservoir, or port, implanted to a patient. Note that even though the sharp cannula 24 of the safety needle inserter of the Figs. 1-4 embodiment is a unitary needle mounted vertically from arm 12, a right angle needle that has a vertical portion and a horizontal portion embedded in arm 12 may also be used as the safety needle inserter of the instant invention. In the case where a right angle needle is used, the distal end of the horizontal portion of the right angle needle may be connected to a catheter, tube or tubing so that fluid may be conveyed to, or removed from, the sharp cannula 24. The sharp cannula may be usable singly, or coaxially with a blunt cannula of the to be discussed infuser component of the instant invention.

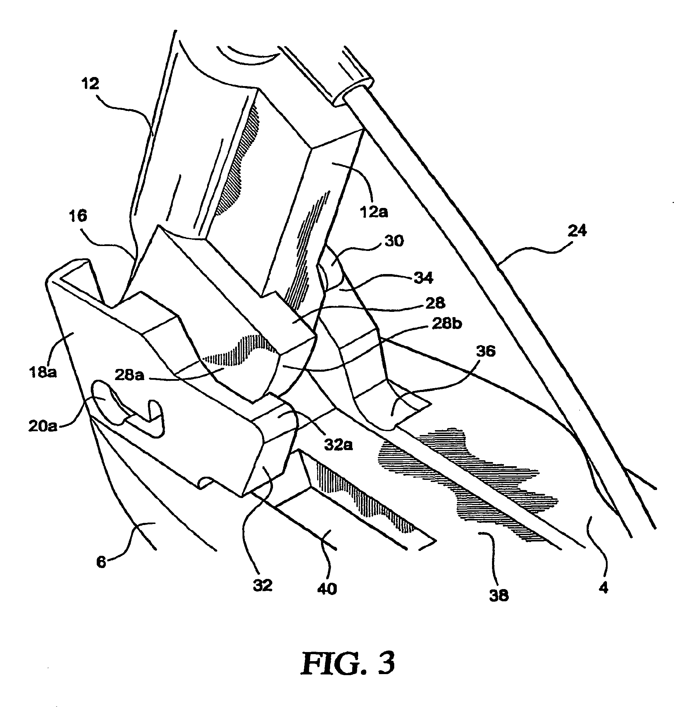

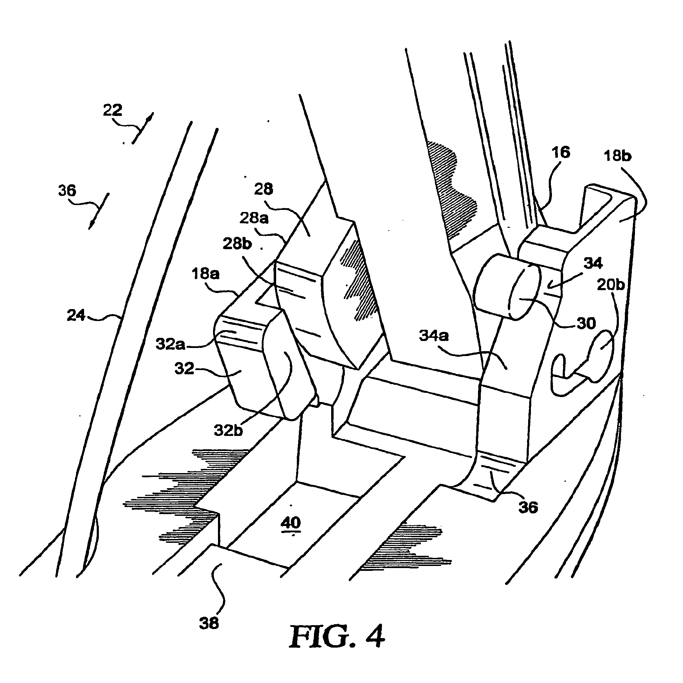

Figs. 3 and 4 illustrate the locking mechanisms at the respective uprights 18a and 18b at distal portion 6 of base 4 and the corresponding coacting locking mechanisms formed at end 16 of arm 12 for preventing further movement of arm 12, once it has been pivotally moved relative to base 4 when tip 26 of sharp cannula 24 is positioned within a bore 42 formed in base 4, best illustrated in the embodiment of the safety needle inserter shown in Fig. 5. Although disclosed as different lock mechanisms, the different portions at both arm 12 and uprights 18 of base 4 may be considered as different parts of a single locking mechanism that cooperatively work together to lock arm 12 in place relative to base 4, and more importantly to fixedly maintain tip 26 of sharp cannula 24 within bore 42 formed at proximal portion 8 of base 4, so as to prevent tip 26 from being exposed or fluid thereon from being splattered, when sharp cannula 24 is withdrawn from the patient

In particular, the lock mechanisms, or the portions of the lock mechanism, as shown In the Fig. 3 and 4 embodiment, comprise a leg 28 and a boss 30 at the respective sides of end 16 of arm 12. The lock mechanisms, or the parts of the lock mechanism, at distal portion 6 of base 4 are configured as a shoulder 32 at upright 18a and a groove or recess 34 at upright 18b.

Due to the Inherent elasticity in the medical plastics that are used for form molding the base and the arm of the safety needle insertion device, when arm 12 is moved along the direction indicated by directional arrow 22, the side surface 28a of leg 28 would bias against the surface 32a of leg 32, as boss 30 may or may not be sliding along surface 34a of groove 34, so that once the foot 28b of leg 28 passes ledge 32b of shoulder 32, shoulder 32 will return to its original position so that latch 32b and foot 28b would bias or coact against each other to prevent any downward movement, as indicated by directional arrow 36, of arm 12. At substantially the same time, once boss 30 reaches groove 34 and biases thereagainst, further upward movement of arm 12 relative to base 4 is prevented.

To enable arm 12 to lie co-planarly on base 4, a notch 36 is provided on base 4 as a rest for boss 32. Further, a channel 38 is molded into base 4 to allow the downward extending portion 12a of arm 12 to fit in alignment to base 4, when arm 12 is in the position vis-a-vis base 4 as shown in Figs. 1 and 2. A void 40 is provided on base 4 to receive leg 28 of arm 12 so that arm 12 could lie substantially along base 4 as shown in Figs. 1 and 2.

Fig. 5 is an illustration of a second embodiment of the safety needle inserter of the instant invention. Other than the overall look of the safety needle inserter and the various parts of the locking mechanisms which coact to lock arm 12 in place relative to base 4, the safety needle inserter of the Fig. 5 embodiment functions in the same way as the embodiment shown in Figs. 1-4. The components that are the same as those of the earlier disclosed embodiment are labeled the same.

As shown more clearly by the partial cut-away view, the tip 26 of sharp cannula 24, when withdrawn from the portal reservoir and the patient, is maintained within a bore 42 that is formed at the proximal portion 8 of base 4. As shown, bore 42 is defined between opening 44 at the underside or lower surface of base 4 and an opening 44a at the upper side or upper surface 46 of base 4. A dam 48 extends from proximal portion 8 of base 4 and serves as an additional safety feature to ensure that the tip 26 of cannula 24 could not be forcibly removed from bore 42. In addition, the top 48a of dam 48 provides a rest stop for arm 12, with end 14 thereof resting against top surface 48a, when needle 26 is axially fitted to the blunt cannula of the infuser. Dam 48 also provides a point for the needle inserter where a user can apply the force needed to insert the sharp cannula into the patient and, as will be described *infra,* penetrate the septum of the portal reservoir implanted to the patient. The same dam structure is provided in the earlier embodiment of the safety needle inserter.

The Fig. 5 embodiment of the safety needle inserter is different from the embodiment shown in Figs. 1-4 in that instead of using separate components for preventing upward and downward movements of arm 12, a pair of bosses 50 provided at the end 16 of arm 12 would bias against the respective front faces 52a of a pair of uprights 52 that are formed at the distal portion of base 4, to prevent further upward movement of arm 12 relative to base 4. A second pair of uprights 54 also extend from base 4 to grasp arm 12, when arm 12 lies longitudinally along base 4 with cannula 24 extending though opening 44. The holding of arm 12 by uprights 54 is by way of corresponding fingers 54a and 54b at the uprights 54. Since uprights 54, along with base 4 and arm 12, are all molded from medical plastics material, when arm 12 is moved upwardly per directional arrow 22, fingers 54b of the uprights 54 would give away until end portion 16 of arm 12 passes both fingers 54a and 54b. At which time uprights 54 would return to their respective original positions so that the lower surface 16a of end portion 16 of arm 12 would rest against the respective inclines 54b of uprights 54, thereby preventing arm 12 from any downward movement as indicated per directional arrow 36.

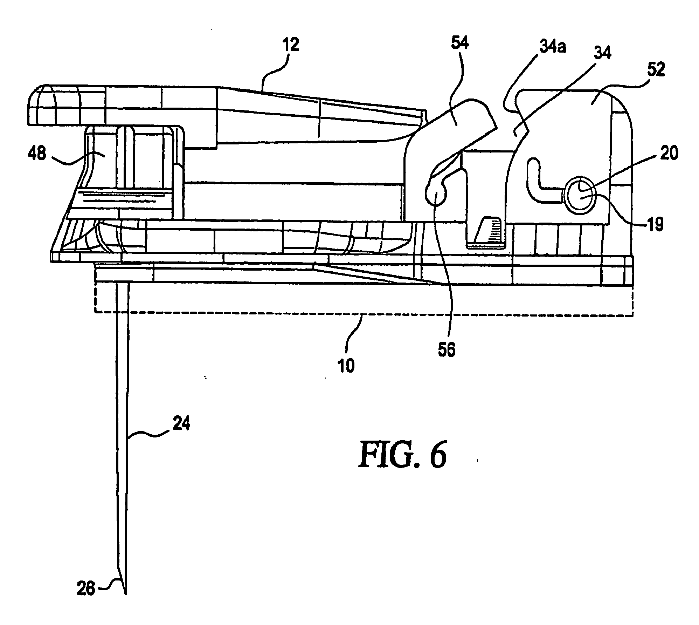

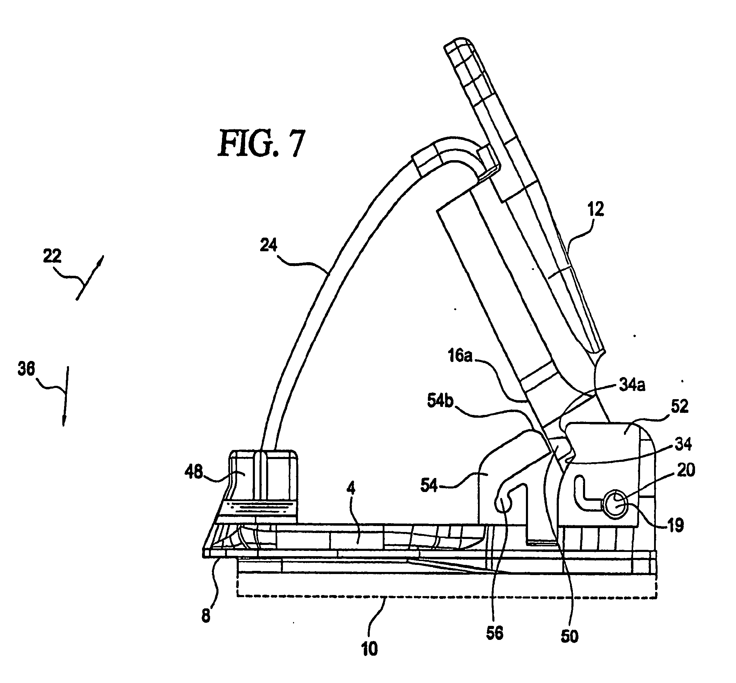

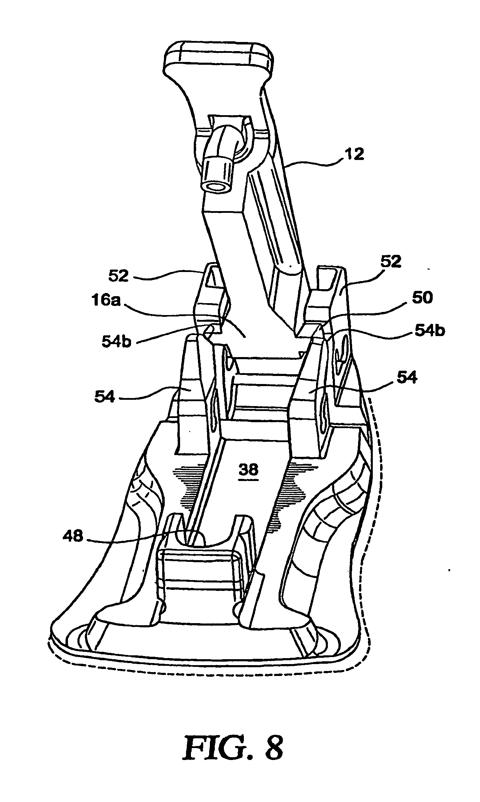

A third embodiment of the safety needle inserter of the instant invention is shown in Figs. 6-8. This embodiment is a modification of the simplified embodiment shown in Fig. 5 in that uprights 54 have been reconfigured to allow additional flexibility by the adding of a notch 56 thereto. As a consequence, when arm 12 is pulled upwards along the direction as indicated by directional arrow 22, each of uprights 54 would tend to flex away, until the underside 16a of arm 12 clears the top edge 54b of the respective uprights 54. At which time, uprights 54 will return to their respective memorized original positions so as to move upper surfaces 54b of uprights 54 under the bottom surface 16a of arm 12 and more specifically those surfaces of the bosses 52, or areas proximate thereto, to thereby prevent downward movement of arm 12 along the direction as indicated by directional arrow 36.

Further, similar to the embodiment shown in Figs. 1-4, respective grooves or recesses 34 are provided at the uprights 52, so that bosses 52 are caught by the front surfaces of the respective recesses 34, designated by 34a, so as to prevent arm 12 from any further upward movement as indicated by directional arrow 22. As before, even though not shown for the sake of clarity, the tip of needle 24 is fixedly retained within a bore at proximal portion 8 of base 4. Note that Figs. 6-8 clearly show that arm 12 is hingedly connected to base 4 by way of its lugs 19 movably journaled to apertures 20 of uprights 52.

As with the other embodiments, a foam layer or pad 10 with an adhesive underside may be provided to the safety needle inserter for enhancing the comfort to the patient, when the inserter is used individually. However, as will be discussed *infra,* the fact that the safety needle inserter is a component of the overall portal access device of the instant invention, and its lower surface does not come into contact with the patient when used with the infuser component, foam 10 is not needed when the safety needle inserter is to be used with the infuser.

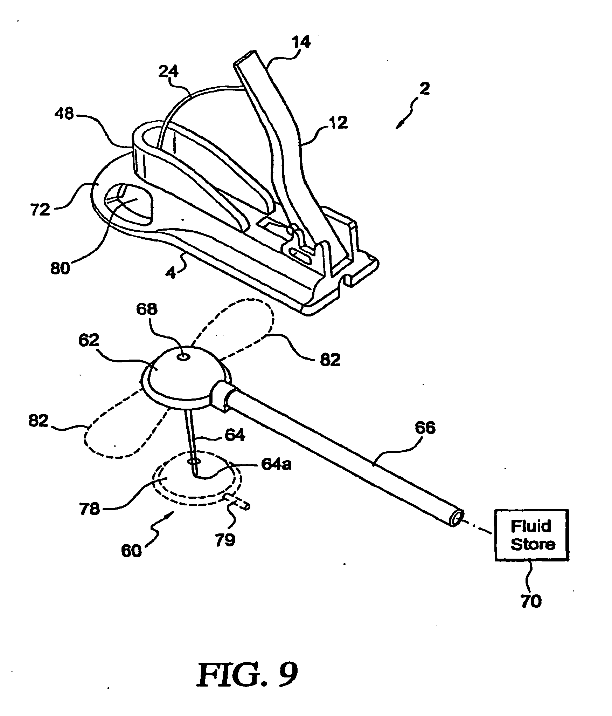

With reference to Figs. 9-12, the portal access device of the instant invention is shown. In particular, a safety needle inserter 2 as described above is used in conjunction with an infuser or a low-profile portal access assembly 60 that includes an infuser body or housing 62, a blunt cannula 64 attached to the underside of body 62 and a catheter or tube 66 that is attached to a side of body 62. As shown in Fig. 9, infuser body 62 is formed as a domed shaped housing or a manifold that has a self-sealing septum 68 at its top in axial alignment with blunt cannula 64. Given that housing 68 is substantially hollow, infuser 60 in essence provides a fluid storage depot through which a communication path between tube 66 and blunt cannula 64 is established.

In operation, tube 66 is connected to a fluid store 70, which may be a syringe or a pump or some other storage device that is adapted for storing fluids or medicaments to be infused to the patient. Alternatively, instead of infusing fluid to the patient, or more particularly to the portal reservoir which is punctured by blunt cannula 64, fluid in the portal reservoir may be retrieved by means of the fluid communication path established between blunt cannula 64 and tube 66 via infuser body 62.

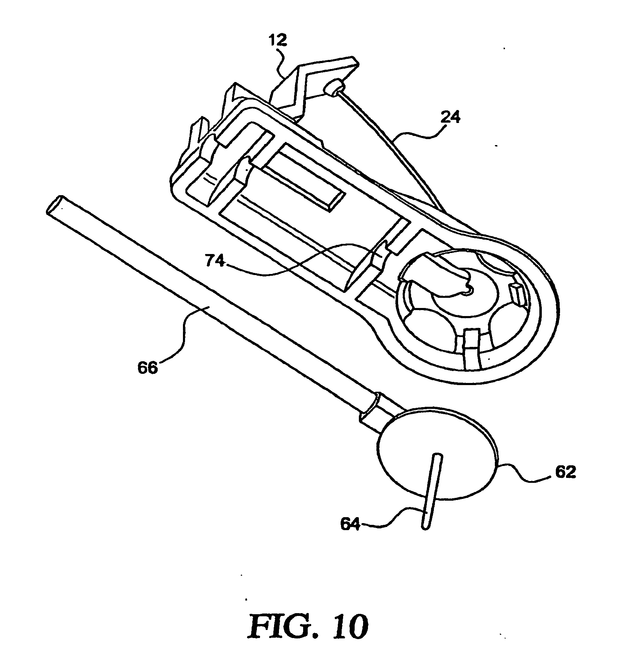

The safety needle inserter component of the portal access device of the instant invention, for the Fig. 9 embodiment, has its proximal portion configured as a dome 72 so that it may be form fitted over infuser body 62. Of course, infuser body 62 may have different dimensions and be configured as different shapes, for example a rectangular shaped housing. In which case, the proximal portion of base 4 of the safety needle inserter would also be rectangularly shaped so that it may form fit over the hypothetical rectangular shaped infuser body. Further, the remainder portion of base 4 may be configured to fit over tube 66, by having a channel 74 formed at its underside as shown by the bottom view of the safety needle inserter in Fig. 10.

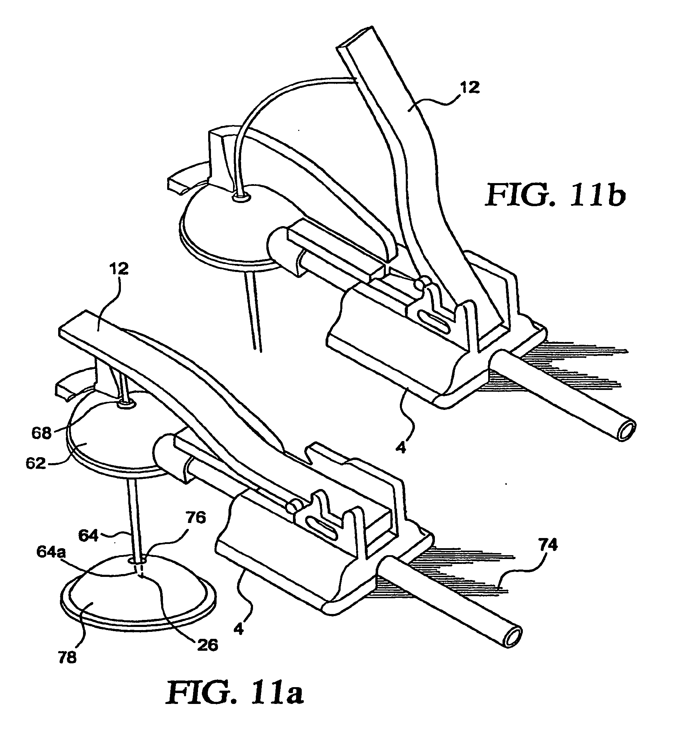

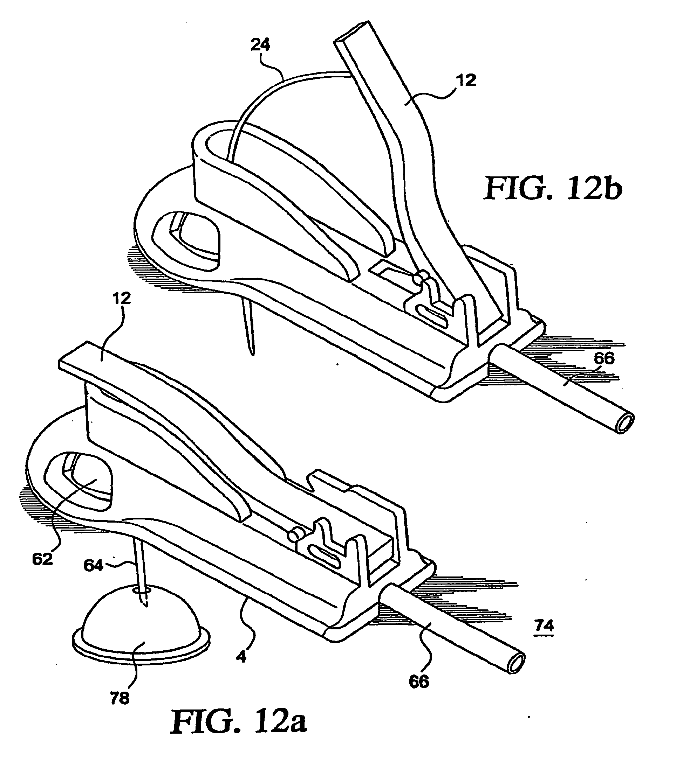

The operation of the safety needle inserter component of the portal access device as shown in Figs. 9 and 10 is the same as that discussed earlier with respect to the various embodiments of the safety needle inserter. When used with infuser assembly 60, safety needle inserter 2 is placed over infuser assembly 60 so that needle or sharp cannula 24 of the needle insertion device 2 is inserted through septum 68 into infuser body 62 and axially through blunt cannula 64 until the tip 26 of sharp cannula 24 protrudes or is exposed beyond the tip 64a of blunt cannula 64. By configuring the proximal portion of needle inserter 2 to form fit over the infuser housing 62, and with arm 12, in particular proximal portion 14 thereof, resting on dam 48 of base 4, a user can readily insert the combination blunt cannula 64/sharp cannula 24 into a patient, designated by 74 in Figs. 11 and 12, so that the tip 26 of cannula 24 may be inserted through a septum opening 36 of a port or portal reservoir 78 implanted in patient 74, per shown in Figs. 11a and 12a. A window 80 may be formed at the proximal portion 8 of base 4 to enable the user to confirm that the dome 72 of the safety needle inserter 2 is appropriately form fitted over infuser body 62, to thereby ensure that cannula 24 be inserted correctly into infuser body 62 and through blunt cannula 64.

Once it is determined that portal reservoir 78 has been properly punctured and connected to infuser body 62 by way of the concentric blunt/sharp cannulas, the safety needle inserter may be removed from the infuser assembly by lifting arm 12 of the needle inserter relative to its base 4, per shown in Figs. 11 b and 12b. Once tip 24 of needle 24 is positioned within the bore of the base, with the respective lock mechanisms coacting at the arm and the base, arm 12 is prevented from further movement both upwardly and downwardly relative to base 4, as per discussion *supra,* so that tip 26 of needle 24 is fixedly maintained or positioned within the bore 42 formed in base 4.

Once arm 12 is locked into place, with tip 26 of the needle 24 properly positioned within bore 42 of base 4, the safety needle inserter may be removed from infuser assembly 60. To ensure that infuser body 62 and tube 66 would remain in place while needle inserter 2 is being removed, infuser body 62 may be provided with a pair of wings 82, either by bonding or molding, so that the user can hold wings 82 against the body or the skin surface of the patient, while lifting safety needle inserter 2 away from infuser assembly 60.

After removal of the safety needle inserter 2, infuser assembly 60 may be secured to the skin surface of the patient, by an adhesive layer preformed at the underside of infuser body 62 or an adhesive tape for example, so that a fluid communication path is continuously established between the portal reservoir 78 implanted in the patient and the fluid store 70. As a consequence, medicaments and other fluids are easily and readily infused to the patient through portal reservoir 78 and its associated catheter or tubing 79. Moreover, infuser assembly 60 provides a long term access to the portal reservoir 78 without further risks of sharps injury, as the safety needle inserter 2 has been removed and no further reinsertion is needed. Furthermore, given that the cannula 64 for infuser assembly 60 is a blunt cannula, when the infuser assembly is removed, there is no risk of injury to the patient or the user, and therefore no sharps protection is required.

Yet furthermore, given that septum 68 of the infuser assembly is self-sealing, infuser assembly 60 may be re-accessed through the self-sealing septum 68, either to provide direct coaxial access to the chamber of the portal reservoir 78, or access to the portal outlet catheter 79, for example by a radially flexible and axially stiff (pushable) wire or rod so that the fluid communication path may be threaded or rodded to clear any obstructions that may have occurred in either the portal reservoir or catheter 79 connected to the portal reservoir though which the medicament is fed to the patient.

Moreover, if a particular type of medicament is required, instead of providing the medicament through the fluid store 70, which feeds to tube 66 of the infuser assembly, a right angle needle insertion device such as that described earlier, or as disclosed In the above-discussed 015 patent, may be used to directly access the Internal chamber of the portal reservoir, either via the blunt cannula of the infuser assembly or separate from the infuser assembly, so that the particular fluid may be fed through the catheter or tubing connected to the horizontal portion of the right angle needle directly into the portal reservoir. Also, the removal of fluid from the portal reservoir may be effected in the reverse manner by suctioning the fluid in the portal reservoir through a right angle needle inserter, by applying suction to the catheter connected to the horizontal portion of the right angle needle while the vertical portion of the right angle needle is in fluid communication with the portal reservoir.

## Claims

1. An apparatus comprising a base (4) having a distal portion (6) and a proximal portion (8), a cannula (24) attached to a first end (14) of an arm (12), a second end (16) of said arm (12) hingedly connected to the distal portion (6) of said base (4) so that said arm (12) is pivotable relative to said base (4) at its second end (16), one opening (44a) at an upper surface of said base (4) and another opening (44) at a lower surface of said base (6) provided at the proximal portion (8) of said base (4) wherethrough said cannula (24) passes a bore (42) defined between said one opening (44a) at the upper surface of said base (4) and said other opening (44) at the lower surface of said base (4); **characterised by** a first lock mechanism (18,34) (50,54) provided at the distal portion (6) of said base and a second lock mechanism (28, 30) (50,16a) provided at the second end (16) of said arm (12), said first and second lock mechanisms coacting to prevent further movement of said arm (12) relative to said base (4) after the tip (26) of said cannula moves past said other opening (44) at the lower surface of said base and is within said bore (42).

2. An apparatus of claim 1, wherein said second lock mechanism comprises a leg (18) extending from the second end (16) of said arm (12) and wherein first lock mechanism (18, 34; 50,54) comprises a shoulder (32) at the distal portion (6) of said base (4), wherein said leg (18) biases against said shoulder (32) to prevent said arm (12) from further downward movement once said arm (12) has been pivoted to a predetermined position whereby the tip (26) of said cannula (24) is positioned within said bore (42).

3. An apparatus of claim 1, wherein said second lock mechanism (28, 30; 50,18a) comprises a boss (34) extending from the second end (16) of said arm (12) and wherein said first lock mechanism (18, 34; 50,54) comprises a groove or recess (34) at the distal portion (60) of said base (4), wherein said boss (34) is caught in and biases against said groove or recess (34) to prevent said arm (12) from further upward movement once said arm (12) has been pivoted to a predetermined position whereby the tip (26) of said cannula (24) is positioned within said bore (42).

4. An apparatus of claim 1, wherein said second lock mechanism (28,30; 50,16a) comprises a leg (18) extending from one side of said arm (12) and a boss (34) extending from another side of said arm (12), and said first lock mechanism (18, 34; 50,54) comprises a shoulder (32) at one side of the distal portion (6) of said base and a groove or recess (34) provided at an upright (18b) at another side of the distal portion (6) of said base (4); and
wherein when said arm (12) is pivoted upwards relative to said base (4) to a predetermined position whereby the tip (26) of said cannula (24) is positioned within said bore, (42) said leg (18) biases against said shoulder (32b) to prevent said arm (12) from downward movement and said boss (30) biases against said groove or recess (34) to prevent said (12) arm from further upward movement so that said arm (12) is locked into said predetermined position relative to said base (4).

5. An apparatus of claim 1, wherein said second end (16) of said arm (12) is hingedly connected to two uprights (52) at the distal portion (6) of said base (4);
wherein said first lock mechanism (18, 34; 50, 54) comprises:
two fingers (50) extending sideways from said arm (12) proximate to its second end (16), each of said fingers (50) having at least two stop surfaces;
wherein said second locking mechanism (28, 30; 50,16a) comprises:
a pair of upright guides (54) each extending from said base (4) to face a corresponding one of said uprights (52), each guide (54) having a notch (56) to which a corresponding one of the fingers (50) from said arm (12) rests when said arm (12) lies horizontally over said base (4), each guide (54) having a guide surface along which the corresponding one finger (50) of said arm (12) moves when said arm (12) is pivoted upwards away from said base (4) and a stop surface (54b) that coacts against one of the stop surfaces of the corresponding one finger (50) when the pivotal movement of said arm (12) positions the tip (26) of said cannula (24) within said bore (42) to thereby prevent said arm from moving in a downward direction (36); and
a recess (34) formed on each front edge of said uprights (52), said recess (34a) coacts against other of the stop surfaces of the corresponding one finger (50) when the pivotal movement of said arm (12) positions the tip (26) of said cannula (24) within said bore (42) to thereby prevent said arm (12) from further upward movement relative to said base (4).

6. An apparatus of claim 1, further comprising a dam (48) at the proximal portion forward of said opening (44) and said bore (42), said dam (48) providing a stop for the first end (4) of said arm (12) for enabling a user to push said cannula (24) into a patient by pressing on said arm (12).

7. An apparatus of claim 1, wherein said cannula (24) comprises a vertical portion extending downwardly from the first end (14) of said arm (12) and a horizontal portion mounted along the length of said arm (12), the tip (26) and a substantial portion of said vertical portion extending beyond said opening (44) of said base (4) prior to use.

8. An apparatus of claim 1, further comprising a tube connected to the horizontal portion of said cannula (24) at the second end (16) of said arm (12) or establishing a fluid path whereby fluid is passable between said tube and the tip (26) of said cannula (24).

9. An apparatus of claim 1, wherein said base (4) has a substantially flat lower surface to enable said base (4) to lie substantially planarly along the skin of a patient, and wherein said bore (42) extends from said other opening (44) substantially flush with the flat lower surface to said one opening (44a) at the top surface of said base (4).

10. An apparatus of claim 1, wherein the proximal portion (8) of said base (4) is dome (72) shaped to enable said base (4) to fittingly cover an infuser.

11. An apparatus of claim 1,
wherein said cannula (24) comprises a right angle cannula mounted to said arm (12), a vertical portion of said cannula (24) including the tip (26) of said cannula (24) extending downwardly from the first end (14) of said arm (12) and passes through said opening (44) of said base (4) before use, a horizontal portion of said cannula (24) mounted along the length of said arm (12);
wherein said first lock mechanism (18, 34; 50, 54) has a first and a second part (32, 34) provided at the distal portion (6) of said base (4); and
wherein a second lock mechanism (28, 40; 50, 16a) has a first and second part (28, 30) provided at the second end (16) of said arm (4);
wherein the respective first parts (28, 32) of said first and second lock mechanisms coact to prevent further movement of said arm (12) relative to said base (4) along one direction after the tip (26) of said cannula (24) is positioned within said bore (42), and
wherein the respective second parts (30, 34) of said first and second lock mechanisms coact to prevent further movement of said arm (12) relative to said base (4) along another direction once the tip (16) of said cannula (24) is positioned within said bore (42).

## Patentansprüche

1. Vorrichtung, die eine Basis (4) mit einem distalen Abschnitt (6) und einem proximalen Abschnitt (8), eine Kanüle (24), die an einem ersten Ende (14) eines Arms (12) angebracht ist, wobei ein zweites Ende (16) des Arms (12) drehbar mit dem distalen Abschnitt (6) der Basis (4) verbunden ist, so dass der Arm (12) in Bezug auf die Basis (4) an seinen zweitem Ende (16) schwenkbar ist, eine Öffnung (44a) an einer oberen Fläche der Basis (4) und eine andere Öffnung (44) an einer unteren Fläche der Basis (6), die an dem proximalen Abschnitt (8) der Basis (4) bereitgestellt ist, umfasst, wobei die Kanüle (24) durch die andere Öffnung (44) durch eine Bohrung (42) verläuft, die zwischen der einen Öffnung (44a) an der oberen Fläche der Basis (4) und der anderen Öffnung (44) an der unteren Fläche der Basis (4) definiert ist; **gekennzeichnet durch** einen ersten Arretiermechanismus (18, 34) (50, 54), der an dem distalen Abschnitt (6) der Basis vorgesehen ist, und einen zweiten Arretiermechanismus (28, 30) (50, 16a), der an dem zweiten Ende (16) des Arms (12) vorgesehen ist, wobei der erste und der zweite Arretiermechanismus zusammenwirken, um eine weitere Bewegung des Arms (12) in Bezug auf die Basis (4) zu verhindern, nachdem die Spitze (26) der Kanüle sich an der anderen Öffnung (44) an der unteren Fläche der Basis vorbei bewegt hat und sich in der Bohrung (42) befindet.

2. Vorrichtung nach Anspruch 1, wobei der zweite Arretiermechanismus einen Fuß (18) umfasst, der sich von dem zweiten Ende (16) des Arms (12) erstreckt, und wobei der erste Arretiermechanismus (18, 34; 50, 54) eine Schulter (32) an dem distalen Abschnitt (6) der Basis (4) umfasst, wobei der Fuß (18) gegen die Schulter (32) vorspannt, um den Arm (12) an einer weiteren Abwärtsbewegung zu hindern, sobald der Arm (12) in eine vorher festgelegte Position geschwenkt ist, wodurch die Spitze (26) der Kanüle (24) in der Bohrung (42) positioniert wird.

3. Vorrichtung nach Anspruch 1, wobei der zweite Arretiermechanismus (28, 30; 50, 18a) einen Buckel (34) umfasst, der sich von dem zweiten Ende (16) des Arms (12) erstreckt, und wobei der erste Arretiermechanismus (18, 34; 50, 54) eine Nut oder Aussparung (34) an dem distalen Abschnitt (60) der Basis (4) umfasst, wobei der Buckel (34) in die Nut oder Aussparung (34) einhakt und gegen diese vorspannt, um den Arm (12) an einer weiteren Aufwärtsbewegung zu hindern, sobald der Arm (12) in eine vorher festgelegte Position geschwenkt ist, wodurch die Spitze (26) der Kanüle (24) in der Bohrung (42) positioniert wird.

4. Vorrichtung nach Anspruch 1, wobei der zweite Arretiermechanismus (28, 30; 50, 16a) einen Fuß (18), der sich von einer Seite des Arms (12) erstreckt, und einen Buckel (34), der sich von der anderen Seite des Arms (12) erstreckt, umfasst und der erste Arretiermechanismus (18, 34; 50, 54) eine Schulter (32) an einer Seite des distalen Abschnitts (6) der Basis und eine Nut oder Aussparung (34), die an einem Pfosten (18b) an der anderen Seite des distalen Abschnitts (6) der Basis (4) vorgesehen ist, umfasst; und
wobei, wenn der Arm (12) in Bezug auf die Basis (4) nach oben in einer vorher festgelegte Position geschwenkt wird, wodurch die Spitze (26) der Kanüle (24) in der Bohrung (42) positioniert wird, der Fuß (18) gegen die Schulter (32b) vorspannt, um den Arm (12) an einer Abwärtsbewegung zu hindern, und der Buckel (30) gegen die Nut oder Aussparung (34) vorspannt, um den Arm (12) an einer weiteren Aufwärtsbewegung zu hindern, so dass der Arm (12) in der vorher festgelegten Position in Bezug auf die Basis (4) arretiert wird.

5. Vorrichtung nach Anspruch 1, wobei das zweite Ende (16) des Arms (12) drehbar mit zwei Pfosten (52) an dem distalen Abschnitt (6) der Basis (4) verbunden ist;
wobei der erste Arretiermechanismus (18, 34; 50, 54) Folgendes umfasst:
zwei Finger (50), die sich seitlich von dem Arm (12) benachbart zu dessen zweitem Ende (16) erstrecken, wobei jeder der Finger (50) mindestens zwei Anschlagflächen aufweist;
wobei der zweite Arretiermechanismus (28, 30; 50, 16a) Folgendes umfasst:
ein Paar senkrechter Führungen (54), die sich jeweils von der Basis (4) erstrecken, um einem entsprechenden der Pfosten (52) zugewandt zu sein, wobei jede Führung (54) eine Einkerbung (56) aufweist, in der ein entsprechender der Finger (50) von dem Arm (12) aufliegt, wenn der Arm (12) horizontal über der Basis (4) liegt, wobei jede Führung (54) eine Führungsfläche, entlang der der entsprechende Finger (50) des Arms (12) sich bewegt, wenn der Arm (12) nach oben von der Basis (4) weg geschwenkt wird, und eine Anschlagfläche (54b) aufweist, die gegen eine der Anschlagflächen des entsprechenden Fingers (50) zusammenwirkt, wenn die Schwenkbewegung des Arms (12) die Spitze (26) der Kanüle (24) in der Bohrung (42) positioniert, um dadurch den Arm daran zu hindern, sich in eine Abwärtsrichtung (36) zu bewegen; und
eine Aussparung (34), die an jeder Vorderkante der Pfosten (52) ausgebildet ist, wobei die Aussparung (34a) gegen eine andere der Anschlagflächen des entsprechenden Fingers (50) zusammenwirkt, wenn die Schwenkbewegung des Arms (12) die Spitze (26) der Kanüle (24) in der Bohrung (42) positioniert, um dadurch den Arm (12) an einer weiteren Aufwärtsrichtung in Bezug auf die Basis (4) zu hindern.

6. Vorrichtung nach Anspruch 1, die weiterhin eine Sperre (48) an dem proximalen Abschnitt vor der Öffnung (44) und der Bohrung (42) umfasst, wobei die Sperre (48) einen Anschlag für das erste Ende (4) des Arms (12) bereitstellt, um einem Benutzer zu ermöglichen, die Kanüle (24) durch Drücken auf den Arm (12) in einen Patienten einzuführen.

7. Vorrichtung nach Anspruch 1, wobei die Kanüle (24) einen vertikalen Abschnitt, der sich von dem ersten Ende (14) des Arms (12) nach unten erstreckt, und einen horizontalen Abschnitt, der entlang der Länge des Arms (12) montiert ist, umfasst, wobei die Spitze (26) und ein wesentlicher Teil des vertikalen Abschnitts sich vor Gebrauch über die Öffnung (44) der Basis (4) hinaus erstrecken.

8. Vorrichtung nach Anspruch 1, die weiterhin einen Schlauch umfasst, der mit dem horizontalen Abschnitt der Kanüle (24) an dem zweiten Ende (16) des Arms (12) verbunden ist oder einen Flüssigkeitsweg herstellt, wodurch Flüssigkeit zwischen dem Schlauch und der Spitze (26) der Kanüle (24) durchlaufen kann.

9. Vorrichtung nach Anspruch 1, wobei die Basis (4) eine im Wesentlichen flache untere Fläche aufweist, um der Basis (4) zu ermöglichen, im Wesentlichen eben entlang der Haut eines Patienten zu liegen, und wobei die Bohrung (42) sich von der anderen Öffnung (44), die im Wesentlichen bündig mit der flachen unteren Fläche ist, zu der einen Öffnung (44a) an der oberen Fläche der Basis (4) erstreckt.

10. Vorrichtung nach Anspruch 1, wobei der proximale Abschnitt (8) der Basis (4) domförmig (72) ist, um der Basis (4) zu ermöglichen, eine Infusionsvorrichtung anliegend abzudecken.

11. Vorrichtung nach Anspruch 1,
wobei die Kanüle (24) eine rechtwinklige Kanüle umfasst, die an dem Arm (12) montiert ist, wobei ein vertikaler Abschnitt der Kanüle (24) die Spitze (26) der Kanüle (24) beinhaltet, die sich von dem ersten Ende (14) des Arms (12) nach unten erstreckt und vor Gebrauch durch die Öffnung (44) der Basis (4) verläuft, wobei ein horizontaler Abschnitt der Kanüle (24) entlang der Länge des Arms (12) montiert ist;
wobei der erste Arretiermechanismus (18, 34; 50, 54) einen ersten und einen zweiten Teil (32, 34) aufweist, die an dem distalen Abschnitt (6) der Basis (4) vorgesehen sind; und
wobei ein zweiter Arretiermechanismus (28, 40; 50, 16a) einen ersten und einen zweiten Teil (28, 30) aufweist, die an dem zweiten Ende (16) des Arms (4) vorgesehen sind;
wobei die jeweiligen ersten Teile (28, 32) des ersten und des zweiten Arretiermechanismus zusammenwirken, um eine weitere Bewegung des Arms (12) in Bezug auf die Basis (4) entlang einer Richtung zu verhindern, nachdem die Spitze (26) der Kanüle (24) in der Bohrung (42) positioniert wurde, und
wobei die jeweiligen zweiten Teile (30, 34) des ersten und des zweiten Arretiermechanismus zusammenwirken, um eine weitere Bewegung des Arms (12) in Bezug auf die Basis (4) entlang einer anderen Richtung zu verhindern, sobald die Spitze (26) der Kanüle (24) in der Bohrung (42) positioniert wurde.

## Revendications

1. Appareil comprenant une base (4) ayant une partie distale (6) et une partie proximale (8), une canule (24) attachée à une première extrémité (14) d'un bras (12), une deuxième extrémité (16) dudit bras (12) étant reliée de manière articulée à la partie distale (6) de ladite base (4) de manière à ce que ledit bras (12) soit capable de pivoter par rapport à ladite base (4) à sa deuxième extrémité (16), une ouverture (44a) à une surface supérieure de ladite base (4) et une autre ouverture (44) à une surface inférieure de ladite base (6) prévues à la partie proximale (8) de ladite base (4) à travers laquelle ladite canule (24) passe dans un alésage (42) défini entre ladite une ouverture (44a) à la surface supérieure de ladite base (4) et ladite autre ouverture (44) à la surface inférieure de ladite base (4) ; **caractérisé par** un premier mécanisme de blocage (18, 34) (50, 54) prévu à la partie distale (6) de ladite base et par un deuxième mécanisme de blocage (28, 30) (50, 16a) prévu à la deuxième extrémité (16) dudit bras (12), lesdits premier et deuxième mécanismes de blocage agissant conjointement de façon à empêcher le déplacement supplémentaire dudit bras (12) par rapport à ladite base (4) après que le bout (26) de ladite canule s'est déplace au-delà de ladite autre ouverture (44) à la surface inférieure de ladite base et se trouve à l'intérieur dudit alésage (42).

2. Appareil selon la revendication 1, dans lequel ledit deuxième mécanisme de blocage comprend une patte (18) s'étendant depuis la deuxième extrémité (16) dudit bras (12) et dans lequel le premier mécanisme de blocage (18, 34 ; 50, 54) comprend un épaulement (32) à la partie distale (6) de ladite base, dans lequel ladite patte (18) exerce une sollicitation contre ledit épaulement (32) pour empêcher ledit bras (12) de se déplacer plus vers le bas une fois que ledit bras (12) a été pivoté dans une position prédéterminée qui fait que le bout (26) de ladite canule (24) est positionné à l'intérieur dudit alésage (42).

3. Appareil selon la revendication 1, dans lequel ledit deuxième mécanisme de blocage (28, 30 ; 50, 18a) comprend un bossage (34) s'étendant depuis la deuxième extrémité (16) dudit bras (12) et dans lequel le premier mécanisme de blocage (18, 34 ; 50, 54) comprend une gorge ou un évidement (34) à la partie distale (60) de ladite base (4), dans lequel ledit bossage (34) est pris dans ladite gorge ou ledit évidement (34) et exerce une sollicitation contre celle-ci ou celui-ci pour empêcher ledit bras (12) de se déplacer plus vers le haut une fois que ledit bras (12) a été pivoté dans une position prédéterminée qui fait que le bout (26) de ladite canule (24) est positionné à l'intérieur dudit alésage (42).

4. Appareil selon la revendication 1, dans lequel ledit deuxième mécanisme de blocage (28, 30 ; 50, 16a) comprend une patte (18) s'étendant depuis un côté dudit bras (12) et un bossage (34) s'étendant depuis un autre côté dudit bras (12), et ledit premier mécanisme de blocage (18, 34 ; 50, 54) comprend un épaulement (32) sur un côté de la partie distale (6) de ladite base et une gorge ou un évidement (34) prévus dans un montant (18b) sur un autre côté de la partie distale (6) de ladite base (4) ; et
dans lequel, lorsque ledit bras (12) est pivoté vers le haut par rapport à ladite base (4) dans une position prédéterminée qui fait que le bout (26) de ladite canule (24) est positionné à l'intérieur dudit alésage (42), ladite patte (18) exerce une sollicitation contre ledit épaulement (32b) afin d'empêcher ledit bras (12) de se déplacer vers le bas et ledit bossage (30) exerce une sollicitation contre ladite gorge ou ledit évidement (34) pour empêcher ledit bras (12) de se déplacer plus vers le haut de manière à ce que ledit bras (12) soit bloqué dans ladite position prédéterminée par rapport à ladite base (4).

5. Appareil selon la revendication 1, dans lequel ladite deuxième extrémité (16) dudit bras (12) est reliée de manière articulée à deux montants (52) à la partie distale (6) de ladite base (4) ;
dans lequel ledit premier mécanisme de blocage (18, 34 ; 50, 54) comprend :
deux doigts (50) s'étendant latéralement depuis ledit bras (12) à proximité de sa deuxième extrémité (16), chacun desdits doigts (50) ayant au moins deux surfaces de butée ;
dans lequel ledit deuxième mécanisme de blocage (28, 30 ;50, 16a,) comprend :
une paire de guides de montants (54) s'étendant chacun à partir de ladite base (4) pour faire face à un montant correspondant desdits montants (52), chaque guide (54) ayant une encoche (56) sur laquelle un doigt correspondant des doigts (50) depuis ledit bras (12) repose lorsque ledit bras (12) est situé horizontalement au-dessus de ladite base (4), chaque guide (54) ayant une surface de guidage le long de laquelle l'un doigt correspondant (50) dudit bras (12) se déplace lorsque ledit bras (12) est pivoté vers le haut en s'écartant de ladite base (4) et une surface de butée (54b) qui agit conjointement contre une des surfaces de butée de l'un doigt correspondant (50) lorsque le mouvement de pivotement dudit bras (12) positionne le bout (26) de ladite canule (24) à l'intérieur dudit alésage (42) pour empêcher ainsi ledit bras de se déplacer dans une direction vers le bas (36) ; et
un évidement (34) formé sur chaque bord avant desdits montants (52), ledit évidement (34a) agissant conjointement contre l'autre des surfaces de butée de l'un doigt correspondant (50) lorsque le mouvement de pivotement dudit bras (12) positionne le bout (26) de ladite canule (24) à l'intérieur dudit alésage (42) afin d'empêcher ainsi ledit bras (12) de se déplacer plus vers le haut par rapport à ladite base (4).

6. Appareil selon la revendication 1, comprenant en outre un barrage (48) à la partie proximale en avant de ladite ouverture (44) et dudit alésage (42), ledit barrage (48) fournissant une butée pour la première extrémité (4) dudit bras (12) pour permettre à un utilisateur de pousser ladite canule (24) dans un patient en appuyant sur ledit bras (12).

7. Appareil selon la revendication 1, dans lequel ladite canule (24) comprend une partie verticale s'étendant vers le bas depuis la première extrémité (14) dudit bras (12) et une partie horizontale montée le long de la longueur dudit bras (12), le bout (26) et une partie importante de ladite partie verticale s'étendant au-delà de ladite ouverture (44) de ladite base (4) avant l'utilisation.

8. Appareil selon la revendication 1, comprenant en outre un tube relié à la partie horizontale de ladite canule (24) à la deuxième extrémité (16) dudit bras (12) ou établissant une voie de passage de fluide, ce qui fait que du fluide peut passer entre ledit tube et le bout (26) de ladite canule (24).

9. Appareil selon la revendication 1, dans lequel ladite base (4) a une surface inférieure essentiellement plate pour permettre à ladite base (4) de reposer essentiellement de manière plane le long de la peau d'un patient, et dans lequel ledit alésage (42) s'étend depuis ladite autre ouverture (44) essentiellement à ras de la surface inférieure plate jusqu'à ladite une ouverture (44a) à la surface supérieure de ladite base (4).

10. Appareil selon la revendication 1, dans lequel la partie proximale (8) de ladite base (4) est en forme de dôme (72) pour permettre à ladite base (4) de couvrir un infuseur de manière ajustée.

11. Appareil selon la revendication 1,
dans lequel ladite canule (24) comprend une canule à angle droit montée sur ledit bras (12), une partie verticale de ladite canule (24) comprenant le bout (26) de ladite canule (24) s'étendant vers le bas depuis la première extrémité (14) dudit bras (12) et passe à travers ladite ouverture (44) de ladite base (4) avant l'utilisation, une partie horizontale de ladite canule (24) étant montée le long de la longueur dudit bras (12) ;
dans lequel ledit premier mécanisme de blocage (18, 34 ; 50, 54) a une première et une deuxième partie (32, 34) prévues à la partie distale (6) de ladite base (4) ; et
dans lequel un deuxième mécanisme de blocage (28, 40 ; 50, 16a) a une première et une deuxième partie (28, 30) prévues à la deuxième extrémité (16) dudit bras (4) ;
dans lequel les premières parties respectives (28, 32) desdits premier et deuxième mécanismes de blocage agissent conjointement pour empêcher le déplacement supplémentaire dudit bras (12) par rapport à ladite base (4) le long d'une direction après que le bout (26) de ladite canule (24) a été positionné à l'intérieur dudit alésage (42), et
dans lequel les deuxièmes parties respectives (30, 34) desdits premier et deuxième mécanismes de blocage agissent conjointement pour empêcher le déplacement supplémentaire dudit bras (12) par rapport à ladite base (4) le long d'une autre direction une fois que le bout (16) de ladite canule (24) est positionné à l'intérieur dudit alésage (42).
